# EUROPEAN PATENT APPLICATION

(11) **EP 2 366 288 A1**
(43) Date of publication of application: **21.09.2011**
(21) Application number: 09829012.5
(22) Date of filing: 18.11.2009
(51) Int. Cl.: A01N 31/02, A01N 25/30, A01N 33/12, A01N 65/36, A01P 3/00, A47L 13/17

(54) **CHEMICAL LIQUID FOR ALCOHOL STERILIZATION SHEET AND ALCOHOL STERILIZATION SHEET CONTAINING SAME**

(30) Priority: 27.11.2008 JP 2008303138
(71) Applicant: Unicharm Corporation, Ehime 799-0111 (JP)
(72) Inventor: BANDOH, Takeshi, Kanonji-shi Kagawa 769-1602 (JP); UEDA, Takahiro, Kanonji-shi Kagawa 769-1602 (JP); ISHIDA, Misaki, Amagasaki-shi Hyogo 660-0095 (JP); ODA, Yoshihito, Amagasaki-shi Hyogo 660-0095 (JP); TERAOKA, Satomi, Amagasaki-shi Hyogo 660-0095 (JP)
(74) Representative: Eke, Philippa Dianne
(86) International application number: PCT/JP2009/069568
(87) International publication number: WO 2010/061764

(57) **Abstract**

The liquid composition of the present invention contains 0.01 to 1 wt% of a nonionic surfactant (a), 0.05 to 1 wt% of a dihydric alcohol having 2 to 4 carbon atoms (b), and 30 to 70 wt% of ethanol (c), the nonionic surfactant (a) is at least one selected from the group consisting of alkyl ether type nonionic surfactants and polyoxyethylene hydrogenated castor oil type nonionic surfactants, the mole number of ethylene oxide per mole of the nonionic surfactant (a) being 20 to 100,and the HLB value of the nonionic surfactant (a) being 13 to 20. According to the present invention, a liquid composition for obtaining an alcohol sterilization sheet, that wipes smoothly, removes grime well, dries quickly after wiping, does not leave wipe marks, does not result in uneven wipe, and does not have a strong alcohol odor, can be obtained.

## Description

### Technical Field

The present invention relates to a liquid composition for an alcohol sterilization sheet and to an alcohol sterilization sheet containing the liquid composition. More specifically, the present invention relates to a liquid composition for an alcohol sterilization sheet for cleaning and sterilizing the surfaces of desks, furniture, floors and the like as well as the surfaces of metal, glass, resin, paint and the like, and to an alcohol sterilization sheet containing the liquid composition.

### Background Art

Recent years have seen more frequent media coverage of food poisoning caused by, for example, 0157 and noroviruses, and there are increasing number of people who pay attention to the hygiene of their living environment. Alcohol has been widely used for sterilizing and disinfecting hands and fingers, since alcohol acts quickly, is highly safe, and barely remains after use. Recently, general consumers are highly likely to use alcohol as a cleaning agent and a sterilizing agent for use on desks, furniture, floors, and like surrounding articles, and increasing demand exists for alcohol as a cleaning agent and a sterilizing agent.

A method used when alcohol is used as a cleaning agent and a sterilizing agent is spraying alcohol with a sprayer or the like onto the target to be wiped and wiping off the fluid with a dry cloth or paper. A more convenient method that has been mainly used in cleaning and sterilizing is using a wet sheet in which a nonwoven fabric or the like is impregnated with an alcohol-containing liquid composition directly. Such alcohol sterilization sheets have features including that they can be immediately used whenever so desired, can be used exclusively on the target to be wiped without spreading a liquid composition to the surroundings, and can effectively sanitize due to the use and the wipe of a liquid composition. However, alcohol sterilization sheets are also problematic in that the liquid composition remains on the target after being wiped; an aggregate derived from grime remains as wipe marks, even after the liquid composition has dried, and a noticeable uneven wipe is created.

As for a liquid composition for an alcohol sterilization sheet that uses alcohol as a principal component, Patent Literature 1 discloses a liquid disinfectant in which butyl parahydroxybenzoate and a moisturizer such as propylene glycol are blended with an alcohol in a specific proportion. This disinfectant is described as barely irritating the skin and bronchial tubes and being unlikely to cause metal corrosion or fiber erosion. Patent Literatures 2 and 3 disclose a sterilizing agent in which a silk protein and other components are blended with an alcohol in a specific proportion. The sterilizing agent is described as being capable of maintaining the sterilizing effect for a long period of time and having an effect to protect the surface of living organisms.

Meanwhile, as for an alcohol sterilization sheet, Patent Document 4 discloses a wiping sheet impregnated with a liquid composition in which a cationic polymeric sterilizing agent is blended with an alcohol in a specific proportion. The sheet is described as being easy to handle and highly effective in sterilizing.

However, the aforementioned liquid compositions and the alcohol sterilization sheet are problematic not only in wipability and grime removal but also in creating noticeable wipe marks and an uneven wipe, especially when dark, glossy desks, glass, and the like are cleaned. In addition, they are also problematic in that when the proportion of alcohol contained is excessive, the alcohol odor is strong, and when the proportion is relatively small, it takes the liquid compositions a long period of time to dry after wiping.

### Prior Art Documents

### Patent Documents

Patent Document 1: Japanese Laid-Open Patent Publication No. H7-252105,
Patent Document 2: Japanese Laid-Open Patent Publication No. 2000-319169
Patent Document 3: Japanese Laid-Open Patent Publication No. 2002-114616
Patent Document 4: Japanese Laid-Open Patent Publication No. 2004-188091
Patent Document 5: Japanese Laid-Open Patent Publication No. H3-90008

### Summary of Invention

### Problems to be Solved by the Invention

An object of the present invention is to provide a liquid composition for an alcohol sterilization sheet, that wipes smoothly, removes grime well, dries quickly after wiping, does not leave wipe marks, does not result in uneven wipe, and does not have a strong alcohol odor, and to provide an alcohol sterilization sheet containing the liquid composition.

### Means for Solving the Problems

Having conducted extensive research to solve the problems described above, the inventors found that a liquid composition for an alcohol sterilization sheet that can achieve the aforementioned object can be obtained by combining a specific nonionic surfactant and a specific polyhydric alcohol with ethanol in a specific proportion, and more preferably further combining a specific antibacterial/bactericidal agent in a specific proportion, and accomplished the present invention.

The liquid composition for an alcohol sterilization sheet of the present invention comprises 0.01 to 1 wt% of a nonionic surfactant (a), 0.05 to 1 wt% of a dihydric alcohol having 2 to 4 carbon atoms (b), and 30 to 70 wt% of ethanol (c), the nonionic surfactant (a) being at least one selected from the group consisting of alkyl ether-type nonionic surfactants and polyoxyethylene hydrogenated castor oil type nonionic surfactants, the mole number of ethylene oxide per mole of the nonionic surfactant (a) being 20 to 100, and the HLB value of the nonionic surfactant (a) being 13 to 20. The component of the balance is an aqueous solvent. The following antibacterial/bactericidal agent (d) and additives are included as necessary.

In an embodiment, the above-mentioned liquid composition further comprises 0.001 to 0.1 wt% of an antibacterial/bactericidal agent (d) , the antibacterial/bactericidal agent (d) being at least one selected from the group consisting of cationic bactericides and grapefruit seed extracts.

The alcohol sterilization sheet of the present invention comprises a base material, the base material being impregnated with the above mentioned liquid composition.

### Effects of Invention

Using the liquid composition for an alcohol sterilization sheet of the present invention, a liquid composition for an alcohol sterilization sheet that wipes smoothly, removes grime well, dries quickly after wiping, does not leave wipe marks, does not result in uneven wipe, and does not have a strong alcohol odor, and an alcohol sterilization sheet containing the liquid composition, can be obtained. This alcohol sterilization sheet is for use in cleaning and sterilizing the surfaces of desks, furniture, floors and the like as well as the surfaces of metal, glass, resin, paint and the like. Mode for Carrying Out the Invention

The liquid composition for an alcohol sterilization sheet of the present invention contains a nonionic surfactant (a), a dihydric alcohol having 2 to 4 carbon atoms (b), and ethanol (c) in a specific proportion. In addition to these components, the liquid composition may further contain an antibacterial/bactericidal agent (d), an additive, or the like, as necessary.

### 1. Components of liquid composition for alcohol sterilization sheet

### (1) Nonionic surfactant (a)

The nonionic surfactant (a) for use in the present invention (hereinafter sometimes referred to as a component a) is at least one member selected from the group consisting of alkyl ether-type nonionic surfactants and polyoxyethylene hydrogenated castor oil-type nonionic surfactants. The mole number of ethylene oxide per mole of the each nonionic surfactant (a) is 20 to 100. The HLB value of each nonionic surfactant (a) is 13 to 20.

The alkyl ether-type nonionic surfactants can be obtained by adding ethylene oxide, propylene oxide, butylene oxide, or the like, preferably ethylene oxide, to a higher fatty alcohol having 8 to 20 carbon atoms, preferably 10 to 18 carbon atoms. A preferable example may be polyoxyethylene (30 mol) lauryl ether or the like, and a commercially available example may be "Nonion K-230" manufactured by NOF Corporation or a similar product. Meanwhile, the polyoxyethylene hydrogenated castor oil-type nonionic surfactants can be obtained by adding ethylene oxide to hydrogenated castor oil. A preferable example may be polyoxyethylene (40 mol) hydrogenated castor oil or the like, and a commercially available example may be "Uniox HC-40" manufactured by NOF Corporation or a similar product. An alkyl ether-type nonionic surfactant is more preferable because the resultant liquid composition does not leave wipe marks or result in uneven wipe.

The mole number of ethylene oxide per mole of the nonionic surfactant (a) is 20 to 100, preferably 20 to 80, and more preferably 20 to 60. When the mole number of ethylene oxide is less than 20, the resultant liquid composition is likely to leave wipe marks and result in uneven wipe. When the molar number of ethylene oxide exceeds 100, it is difficult to formulate the liquid composition and the liquid composition dries slowly after wiping.

The HLB value of the nonionic surfactant (a) is 13 to 20 and preferably 13 to 18. When the HLB is less than 13, the resultant liquid composition is likely to leave wipe marks and result in uneven wipe.

The liquid composition of the present invention contains the nonionic surfactant (a) in a proportion of 0.01 to 1 wt%, preferably 0.01 to 0.7 wt%, and more preferably 0.03 to 0.5 wt%. When the proportion is less than 0.01 wt%, the resultant liquid composition results in insufficient grime removal, leaves wipe marks, produces a noticeable uneven wipe, and gives a strong alcohol odor. When the proportion exceeds 1 wt%, the resultant liquid composition dries slowly after wiping and leaves wipe marks.

### (2) Dihydric alcohol having 2 to 4 carbon atoms (b)

The dihydric alcohol having 2 to 4 carbon atoms (b) for use in the present invention (hereinafter sometimes referred to as a component b) is a water-soluble compound having 2 hydroxyl groups per molecule. In the case of a dihydric alcohol having 5 or more carbon atoms, the resultant liquid composition dries slowly after wiping and has a strong alcohol odor. Examples of the dihydric alcohol having 2 to 4 carbon atoms include ethylene glycol, propylene glycol, 1,3-buthylene glycol, diethylene glycol, and the like. Propylene glycol is particularly preferable because the resultant liquid composition dries quickly after wiping and does not have a strong alcohol odor.

The dihydric alcohol having 2 to 4 carbon atoms (b) is contained in the liquid composition of the present invention in a proportion of 0.05 to 1 wt%, preferably 0.1 to 0.7 wt%, and more preferably 0.3 to 0.7 wt%. When the proportion is less than 0.05 wt%, the resultant liquid composition leaves wipe marks and results in a noticeable uneven wipe. When the proportion exceeds 1 wt%, the resultant liquid composition dries extremely slowly after wiping.

### (3) Ethanol (c)

Ethanol (c) for use in the present invention (hereinafter sometimes referred to as a component c) may be synthetically obtained or obtained through fermentation, and ethanol that is generally commercially distributed as 95% first grade or 99% special grade is usable.

Ethanol (c) is contained in the liquid composition of the present invention in a proportion of 30 to 70 wt%, preferably 35 to 65 wt%, and more preferably 40 to 60 wt%. When the proportion is less than 30 wt%, not only does the resultant liquid composition exhibit little fast-acting bactericidal properties, but also dries extremely slowly after wiping. When the proportion exceeds 70 wt%, the resultant liquid composition dries excessively quickly, resulting in impaired stability and wipability and in strong alcohol odor.

### (4) Antibacterial/bactericidal agent (d)

The liquid composition of the present invention, in order to exhibit a greater sterilizing effect without impairing its effects, i.e., smooth wiping, good grime removal, quick drying after wiping, no remaining wipe marks, no uneven wipe, and no strong alcohol odor, may preferably contain an antibacterial/bactericidal agent (d) (hereinafter sometimes referred to as a component d).

The antibacterial/bactericidal agent (d) for use in the present invention is at least one member selected from the group consisting of cationic bactericidal agents and grapefruit seed extracts.

Cationic bactericidal agents are compounds having a quaternized nitrogen atom in the molecule and have bactericidal properties. Examples of cationic bactericidal agents include benzalkonium chloride, benzethonium chloride, didecyldimethylammonium chloride, cetylpyridinium chloride, and the like.

Grapefruit seed extracts are extracts obtained by performing extraction on seeds of grapefruit (*Citrus paradisi Macf*), which belongs to the *Rutaceae* family, using various solvents, and the extracts refer to the components other than the solvents (extraction solvents, dilution solvents, etc.). For a grapefruit seed extract, commercially available products such as "Desfan-10" (manufactured by Teikku Fine KK) and "Calfa" (manufactured by Calfa Chemical Co., Ltd.) can be used. The "Desfan-10" contains 90.5 wt% of water and 6.25 wt% of glycerol as solvents and thus 3.25 wt% of grapefruit seed extract is contained.

It is disclosed that grapefruit seed extracts have antibacterial properties in Patent Literature 5. In particular, an extract obtained by performing extraction on seeds of unripe grapefruits is preferable in terms of, for example, antibacterial property, stability, and user experience of the resultant liquid composition. For example, the grapefruit seed extract is produced as follows.

First, grapefruit seeds are subjected to pulverization, drying, or like processing as necessary Grapefruits can be used irrespective of their place of origin. Next, seeds or a pulverized, dried, or similarly processed product thereof are subjected to extraction by being immersed in water, an alcohol, a hydrocarbon, an ester, a ketone, an ether, a halogenated hydrocarbon, or a mixed solvent thereof, or being immersed and thermally refluxed therein. Water or an alcohol is preferably used as an extraction solvent. As for the alcohol, both monohydric alcohols and polyhydric alcohols can be used. Water, ethanol, isopropyl alcohol, propylene glycol, 1,3-buthylene glycol, glycerol, or a mixed solvent thereof is more preferable, and water, glycerol, or a mixed solvent thereof is particularly preferable.

When the antibacterial/bactericidal agent (d) is contained in the liquid composition of the present invention, the amount thereof is 0.001 to 0.1 wt%, preferably 0.003 to 0.1 wt%, and more preferably 0.005 to 0.1 wt%. When the amount is less than 0.001 wt%, a sufficient effect of addition is not obtained, and when the amount exceeds 0.1 wt%, the resultant liquid composition is likely to leave wipe marks.

### (5) Additives and other components

The liquid composition for an alcohol sterilization sheet of the present invention may contain an additive that is usually used in cosmetics, cleaning agents, or the like, in such a proportion that the effects of the present invention are not impaired. Examples of such additives include hydrocarbon oils such as liquid paraffin, liquid isoparaffin, squalane, vaseline, and solid paraffin; natural fats and oils such as beef tallow, lard, and fish oil; synthetic triglycerides such as glyceryl tri(2-ethylhexanoate); ester oils such as isopropyl myristate, isopropyl palmitate, cetyl palmitate, ethyl oleate, oleyl oleate, and octyldodecyl myristate; waxes such as beeswax and carnauba wax; silicone derivatives such as linear or cyclic dimethyl polysiloxane and polyether-modified dimethyl polysiloxane; oily bases such as ceramide, cholesterol, protein derivatives, lanolin, lanolin derivatives, and lecithin; nonionic surfactants other than the essential components of the present invention, such as polyoxyethylene alkyl ether, polyethylene glycol fatty acid ester, polyoxypropylene alkyl ether, polypropylene glycol fatty acid ester, sorbitan fatty acid ester, polyoxyethylene hydrogenated castor oil, polyglycerol fatty acid ester, glycerol monofatty acid ester, alkyl polyglucoside, polyoxyethylene polyoxypropylene block polymer, and alkanolamide; anionic surfactants such as soap, sulfuric acid alkyl ester salt, sulfuric acid alkyl ether ester salt, α-olefinsulfonic acid salt, acylmethyltaurine salt, acylglutamic acid salt, acylglycine salt, acylsarcosine salt, acylisethionic acid salt, carboxylic acid alkyl ether salt, sulfuric acid amide ether ester salt, and phosphoric acid alkyl ester salt; amphoteric surfactants such as alkyldimethylaminoacetic acid betaine, amidoamino acid salt, and alkyliminodiacetic acid salt; semi-polar surfactants such as alkylamine oxide and polyoxyethylene alkylamine oxide; cationic surfactants other than the essential components of the present invention such as alkyltrimethylammonium chloride and dialkyldimethylammonium chloride; hydrochlorides and acetates of alkylamine or amidoamine; powders of inorganic substances such as talc, kaolin, sericite, mica, vermiculite, magnesium carbonate, calcium carbonate, diatomaceous earth, magnesium silicate, calcium silicate, aluminum silicate, barium silicate, strontium silicate, barium sulfate, metal tungstate, silica, zeolite, hydroxyapatite, boron nitride, and ceramics; powders of organic substances such as crystalline cellulose, polyethylene, and polytetrafluoroethylene; inorganic pigments such as titanium oxide, zinc oxide, red iron oxide (Indian red), loess, carbon black, cobalt violet, chromium oxide, and ultramarine; pearlescent pigments such as titanium oxide-coated mica, argentine, and colored titanium oxide-coated mica; metal powder pigments such as aluminium powder and copper powder; cosmetics pigments such as red no. 201, red no. 227, orange no. 204, yellow no. 205, and blue no. 404; food additive pigments such as red no. 3, red no. 106, yellow no. 4, yellow no. 5, and blue no. 1; natural pigments such as zirconium, chlorophyll, and ß-carotene; water-soluble polymers such as alginic acid, carboxyvinyl polymer, carboxymethyl cellulose, hydroxypropylmethyl cellulose, xanthan gum, and hyaluronic acid; inorganic or organic salts such as magnesium sulfate, sodium chloride, and sodium citrate; pH adjustors such as acids and alkalis; bactericidal agents other than the essential components of the present invention; chelating agents; antioxidants; ultraviolet absorbers; natural extracts derived from animals and plants; flavoring agents; and the like.

As for the solvent of the liquid composition of an alcohol sterilization sheet of the present invention, solvents in which the aforementioned components can dissolve or disperse and which are harmless to the human body are used. For example, an aqueous solvent such as water (purified water or the like) may be used.

### 2. Liquid composition for alcohol sterilization sheet and alcohol sterilization sheet containing it

The liquid composition for an alcohol sterilization sheet of the present invention contains the aforementioned nonionic surfactant (a), the dihydric alcohol having 2 to 4 carbon atoms (b), the ethanol (c), and an aqueous solution, and the liquid composition may contain the antibacterial/bactericidal agent (d), an additive, and the like, as necessary. Usually, the liquid composition for an alcohol sterilization sheet of the present invention is prepared by dissolving or dispersing the nonionic surfactant (a), the dihydric alcohol having 2 to 4 carbon atoms (b), and the ethanol (c) in an aqueous solvent such as purified water in the aforementioned specific proportion. Moreover, the antibacterial/bactericidal agent (d) and an additive may be dissolved or dispersed in the liquid composition in a specific proportion as necessary.

The liquid composition for an alcohol sterilization sheet of the present invention is used in the form of an alcohol sterilization sheet in which a base material is impregnated with the liquid composition. Examples of the base material include nonwoven fabrics made from natural fiber or synthetic fiber, paper, and the like. Specifically, a spunlaced nonwoven fabric having high wet strength formulated with one or more of hydrophilic fibers such as rayon and pulp and synthetic fibers such as polyester, polyethylene, and polypropylene, or a like fabric, is used. The size of a sheet used as the base material is suitably determined according to the site to which the sheet is applied or according to the container or packaging in which the sheet is accommodated, and usually it is about 150×200 mm. 100 parts by weight of the sheet is impregnated with about 100 to 300 parts by weight of the liquid composition of the present invention, thus giving the alcohol sterilization sheet.

The alcohol sterilization sheet prepared with the liquid composition of the present invention is accommodated in a container to prevent drying. It is preferable that such a container is a relatively hard container that is made from, for example, a synthetic resin such as polyethylene or polypropylene and has a slot with a lid that serves to seal the entire container such that a portion of an alcohol sterilization sheet protruding therefrom does not dry. Alternatively, the alcohol sterilization sheet may be accommodated in a liquid-impermeable film packaging in which pieces of the alcohol sterilization sheet are stacked one on top of the other (e.g., as an embodiment for use as a refill). In addition, the packaging in which the alcohol sterilization sheets stacked one after another are accommodated may be used in the form of a refill, i.e., the packaging is placed in a hard container and used. Specifically, the method described in Japanese Laid-Open Patent Publication No. 2000-79074 and other methods can be adopted.

### Examples

Next, the present invention will now be described in more detail by way of examples.

Liquid compositions prepared in Examples and Comparative Examples below were evaluated according to the following methods (1) to (5). The alcohol sterilization sheets used in the evaluations below were prepared by cutting a spunlaced nonwoven fabric having a weight of 40 g/m² into sheets each having a size of 150×200 mm and then impregnating 100 parts by weight of the nonwoven fabric (sheet) with 280 parts by weight of a liquid composition.

### (1) Wipability and grime removal

15 female and 5 male panelists ranging from 25 to 52 years old wiped a desk, furniture, or the like, with the alcohol sterilization sheets and made a judgment on wipability and grime removal as follows:
2 points: when a panelist felt that wiping was smoothly performed and grime was removed with wiping lightly
1 point: when a panelist felt that wiping was performed slightly less smoothly or it was slightly more difficult to remove grime
0 point: when a panelist felt that wiping was not performed smoothly or it was clearly difficult to remove grime

The scores from the 20 panelists were averaged and the following criterion was used to evaluate wipability and grime removal.
○: Good wipability and grime removal (average score of 1.5 or greater)
×: Poor wipability and grime removal (average score of less than 1.5)

### (2) Speed of drying after wiping

15 female and 5 male panelists ranging from 25 to 52 years old wiped a desk, furniture, or the like with the alcohol sterilization sheets and made a judgment on the extent of drying as follows:
2 points: when a panelist felt that a sheet dried quickly after wiping
1 point: when a panelist felt that a sheet dried slightly more slowly after wiping
0 point: when a panelist felt that a sheet dried slowly after wiping

The scores from the 20 panelists were averaged and the following criterion was used to evaluate the speed of drying.
○: dried quickly after wiping (average score of 1.5 or greater)
×: dried slowly after wiping (average score of less than 1.5)

### (3) Wipe mark

15 female and 5 male panelists ranging from 25 to 52 years old wiped a desk, furniture, or the like with the alcohol sterilization sheets and made a judgment on wipe mark as follows:
2 points: when a panelist felt that no wipe marks were left
1 point: when a panelist felt that slight wipe marks were left
0 point: when a panelist felt that noticeable wipe marks were left

The scores from the 20 panelists were averaged and the following criterion was used to evaluate wipe marks.
○: No wipe marks left (average score of 1.5 or greater)
×: Wipe marks left (average score of less than 1.5)

### (4) Uneven wipe

15 female and 5 male panelists ranging from 25 to 52 years old wiped a desk, furniture, or the like with the alcohol sterilization sheets and made a judgment on uneven wipe as follows:
2 points: when a panelist felt that no uneven wipe was created
1 point: when a panelist felt that a slight uneven wipe was created
0 point: when a panelist felt that a noticeable uneven wipe was created

The scores from the 20 panelists were averaged and the following criterion was used to evaluate an uneven wipe.
○: No uneven wipe produced (average score of 1.5 or greater)
×: Uneven wipe produced (average score of less than 1.5)

### (5) Alcohol odor

15 female and 5 male panelists ranging from 25 to 52 years old wiped a desk, furniture, or the like with the alcohol sterilization sheets and made a judgment on alcohol odor as follows:
2 points: when a panelist felt that an alcohol odor was insignificant
1 point: when a panelist felt that an alcohol odor was slightly more significant
0 point: when a panelist felt a strong alcohol odor

The scores from the 20 panelists were averaged and the following criterion was used to evaluate alcohol odor.
○: No strong alcohol odor (average score of 1.5 or greater)
× : Strong alcohol odor (average score of less than 1.5)

### Examples 1 to 7

The components shown in Table 1 were mixed in the proportions shown in Table 1 to give liquid compositions for an alcohol sterilization sheet (these liquid compositions are referred to as liquid compositions 1 to 7). Using the liquid compositions 1 to 7 thus obtained, (1) wipability and grime removal, (2) wipe mark, (3) uneven wipe, (4) alcohol odor, and (5) the speed of drying after wiping were evaluated according to the test methods described above. Table 1 shows the results.

**Table 1**

| | | | Example | | | | | | |
|---|---|---|---|---|---|---|---|---|---|
| | | | 1 | 2 | 3 | 4 | 5 | 6 | 7 |
| | | | Liquid composition 1 | Liquid composition 2 | Liquid composition 3 | Liquid composition 4 | Liquid composition 5 | Liquid composition 6 | Liquid composition 7 |
| | (a) | Polyoxyethylene (30 mol) lauryl ether (HLB: 17.5) | 0.05 | - | - | 0.03 | 0.1 | 0.03 | 0.05 |
| | | Polyoxyethylene (40 mol) hydrogenated castor oil (HLB: 13.3) | - | 0.1 | 0.08 | - | - | - | - |
| | (b) | Propylene glycol | 0.5 | 1 | 0.5 | 0.5 | 0.3 | 0.1 | 0.5 |
| | (c) | 95% First-grade ethanol | 50 | 50 (47.5) | 50 (47.5) | 50 (47.5) | 50 (47.5) | 40 (38) | 60 (57) |
| Component (wt%) | (d) | Benzalkonium chloride | 0.05 | 0.05 | 0.05 | - | 0.005 | - | 0.005 |
| | | Cetylpyridinium chloride | - | - | - | - | - | 0.02 | 0.02 |
| | | Grapefruit seed extract^{(note 1)} (3.25% active component) | - | - | - | 0.1 (0.003) | 0.1 (0.003) | - | 0.1 (0.003) |
| | Others | Polyoxyethylene (11 mol) polyoxypropylene (4 mol) lauryl ether^{(note2)} (HLB: 11) | - | - | - | - | - | - | - |
| | | Polyoxyethylene (2 mol) stearylamine^{(note3)} | - | - | - | - | - | - | - |
| | | 1,3-Buthylene glycol | - | - | - | - | - | - | - |
| | | Purified water | Balance | Balance | Balance | Balance | Balance | Balance | Balance |
| | Total | | 100 | 100 | 100 | 100 | 100 | 100 | 100 |
| Evaluation result (score) | Wipability and grime removal | | ○ (1.8) | ○ (1.8) | ○ (1.7) | ○ (1.8) | ○ (1.9) | ○ (1.6) | ○ (2) |
| | Speed of drying after wiping | | ○ (2) | ○ (1.6) | ○ (1.7) | ○ (1.9) | ○ (1.8) | ○ (1.6) | ○ (2) |
| | Wipe mark | | ○ (2) | ○ (1.6) | ○ (1.6) | ○ (1.8) | ○ (1.8) | ○ (1.8) | ○ (1.8) |
| | Uneven wipe | | ○ (1.9) | ○ (1.7) | ○ (1.6) | ○ (1.8) | ○ (1.8) | ○ (1.8) | ○ (1.8) |
| | Alcohol odor | | ○ (1.7) | ○ (1.8) | ○ (1.7) | ○ (1.8) | ○ (1.9) | ○ (1.9) | ○ (1.7) |

| | | | | | | | | | |
|---|---|---|---|---|---|---|---|---|---|
| Note 1: "Desfan-10" (manufactured by Teikku Fine KK) Note 2: "Nonion TA-411" (manufactured by NOF Corporation) Note 3: "Nymeen 5202" (manufactured by NOF Corporation) | | | | | | | | | |

### Comparative Examples 1 to 7

The components shown in Table 2 were mixed in the proportions shown in Table 2 to give liquid compositions for an alcohol sterilization sheet (these liquid compositions are referred to as liquid compositions 8 to 14). The liquid compositions 8 to 14 thus obtained were evaluated according to the test methods for (1) to (5) above. Table 2 shows the results.

**Table 2**

| | | | Comparative Example | | | | | | |
|---|---|---|---|---|---|---|---|---|---|
| | | | 1 | 2 | 3 | 4 | 5 | 6 | 7 |
| | | | Liquid composition 8 | Liquid composition 9 | Liquid composition 10 | Liquid composition 11 | Liquid composition 12 | Liquid composition 13 | Liquid composition 14 |
| | (a) | Polyoxyethylene (30 mol) lauryl ether (HLB: 17.5) | 6 | 0.6 | 0.05 | 0.05 | - | - | 0.05 |
| | | Polyoxyethylene (40 mol) hydrogenated castor oil (HLB: 13.3) | - | 0.5 | - | - | - | - | - |
| | (b) | Propylene glycol | 0.5 | 0.5 | - | 1.2 | 0.3 | 0.5 | - |
| | (c) | 95% First-grade ethanol | 50 (47.5) | 50 (47.5) | 50 (47.5) | 50 (47.5) | 50 (47.5) | 40 (38) | 60 (57) |
| Component (wt%) | (d) | Benzalkonium chloride | 0.05 | 0.05 | 0.05 | - | 0.005 | 0.05 | 0.005 |
| | | Cetylpyridinium chloride | - | - | - | - | - | - | 0.02 |
| | | Grapefruit seed extract^{(note1)} (3.25% active component) | - | - | - | 0.1 (0.003) | 0.1 (0.003) | - | 0.1 (0.003) |
| | Others | Polyoxyethylene (11 mol) polyoxypropylene (4 mol) lauryl ether(^{note2)} (HLB: 11) | - | - | - | - | 0.1 | - | - |
| | | Polyoxyethylene (2 mol) stearylamine^{(note3)} | - | - | - | - | - | 0.05 | - |
| | | 1,3-Buthylene glycol | - | - | - | - | - | - | 0.5 |
| | | Purified water | Balance | Balance | Balance | Balance | Balance | Balance | Balance |
| | Total | | 100 | 100 | 100 | 100 | 100 | 100 | 100 |
| Evaluation result (score) | Wipability and grime removal | | × (0.8) | ○ (1.7) | ○ (1.6) | ○ (1.8) | ○ (1.7) | × (1.3) | ○ (1.8) |
| | Speed of drying after wiping | | ○ (1.9) | × (1) | ○ (2) | × (1.3) | ○ (1.5) | × (1.2) | × (1.3) |
| | Wipe mark | | × (0.2) | × (1.2) | × (1.2) | ○ (1.6) | × (0.8) | ○ (1.8) | × (1) |
| | Uneven wipe | | × (0.5) | ○ (1.5) | × (1.4) | ○ (1.7) | × (1.2) | ○ (1.8) | × (1.2) |
| | Alcohol odor | | × (1.2) | ○ (1.8) | ○ (1.7) | ○ (1.8) | ○ (1.6) | × (1.3) | × (1.4) |

| | | | | | | | | | |
|---|---|---|---|---|---|---|---|---|---|
| Note 1: "Desfan-10" (manufactured by Teikku Fine KK) Note 2: "Nonion TA-411" (manufacture by NOF Corporation) Note 3: "Nymeen S202" (manufactured by NOF Corporation) | | | | | | | | | |

As is clear from the results shown in Table 1, the liquid compositions for an alcohol sterilization sheet 1 to 7 of Examples 1 to 7 all wiped smoothly, removed grime well, dried quickly after wiping, did not leave wipe marks, did not result in uneven wipe, and did not have a strong alcohol odor.

On the other hand, as is clear from the results shown in Table 2, sufficient effects were not obtained from the liquid compositions for a cleaning wet wipe 8 to 14 of Comparative Examples 1 to 7. That is, Comparative Example 1 did not contain the component (a) and thus resulted in insufficient wipability and grime removal, left wipe marks, resulted in uneven wipe, and had a strong alcohol odor. Comparative Example 2 contained the component (a) in a proportion exceeding the range of the present invention and thus dried quickly after wiping and left wipe marks. Comparative Example 3 did not contain the component (b) and thus left wipe marks and resulted in uneven wipe. Comparative Example 4 contained the component (b) in a proportion exceeding the range of the present invention and thus dried slowly after wiping. Comparative Examples 5 and 6 used surfactants different from the component (a). Comparative Example 5 resulted in insufficient wipability and grime removal, dried slowly after wiping, and had a strong alcohol odor. Comparative Example 6 dried slowly after wiping, left wipe marks, resulted in uneven wipe, and had a strong alcohol odor. Comparative Example 7 used a polyhydric alcohol different from the component (b) and thus dried quickly after wiping, left wipe marks, resulted in uneven wipe, and had a strong alcohol odor.

### Industrial Applicability

Use of the liquid composition for an alcohol sterilization sheet of the present invention provides an alcohol sterilization sheet. This alcohol sterilization sheet wipes smoothly, removes grime well, dries quickly after wiping, does not leave wipe marks, does not result in uneven wipe, and does not have a strong alcohol odor. This alcohol sterilization sheet is for use in cleaning and sterilizing the surfaces of desks, furniture, floors and the like as well as the surfaces of metal, glass, resin, paint and the like.

## Claims

1. A liquid composition for an alcohol sterilization sheet, comprising:
0.01 to 1 wt% of a nonionic surfactant (a),
0.05 to 1 wt% of a dihydric alcohol having 2 to 4 carbon atoms (b), and
30 to 70 wt% of ethanol (c),
the nonionic surfactant (a) being at least one selected from the group consisting of alkyl ether-type nonionic surfactants and polyoxyethylene hydrogenated castor oil-type nonionic surfactants,
the mole number of ethylene oxide per mole of the nonionic surfactant (a) being 20 to 100, and
the HLB value of the nonionic surfactant (a) being 13 to 20.

2. The liquid composition according to claim 1, further comprising 0.001 to 0.1 wt% of an antibacterial/bactericidal agent (d),
the antibacterial/bactericidal agent (d) being at least one selected from the group consisting of cationic bactericides and grapefruit seed extracts.

3. An alcohol sterilization sheet comprising a base material, the base material being impregnated with the liquid composition of claim 1 or 2.
